# EUROPEAN PATENT SPECIFICATION

(11) **EP 1 902 680 B1**
(45) Date of publication and mention of the grant of the patent: **10.11.2010**
(21) Application number: 07115585.7
(22) Date of filing: 04.09.2007
(51) Int. Cl.: A61B 17/68, A61B 17/82, A61B 17/00, A61B 17/06, A61B 17/84

(54) **A bioabsorbable elongated member**
Verlängertes bioabsorbierbares Element
Élément allongé bioabsorbable

(30) Priority: 20.09.2006 FI 20065574
(43) Date of publication of application: 26.03.2008
(73) Proprietor: BIORETEC OY, 33720 Tampere (FI)
(72) Inventor: Allinniemi, Timo, 33880 Lempäälä (FI); Törmälä, Pertti, 33300 Tampere (FI); Partio, Esa, 01190 Sipoo (FI)
(74) Representative: Hakola, Unto Tapani

(56) References cited:
- EP-A- 1 752 115
- WO-A-99/51159
- WO-A-2006/039296
- JP-A- 2002 201 941
- US-A1- 2005 171 547
- US-A1- 2007 027 475
- US-B1- 6 296 659

## Description

### FIELD OF INVENTION

The present invention relates to a bioabsorbable elongated member which may be used in a bone and/or tissue fixation.

### BACKGROUND OF THE INVENTION

In bone surgery it is well known to use metallic tension band wires in the internal fixation of bone fractures, osteotomies and pseudarthroses. A typical tension band wire is a flexible metallic wire with a diameter of about 1 mm. The clinical use of tension band wires is described e.g. in M.E. Müller et al., "Manual of Internal Fixation", Springen-Verlag, Berlin Heidelberg New York, 1979, pages 42-47. However, tension band wiring has several drawbacks. Because of the high tensile modulus of metallic wires, the tightening of wire too much can lead to bone fractures or to necrosis under the wire. Also the knotting of the wire loop creates a bulky knot which can irritate tissues, especially subcutaneous tissue on a bone, which can lead even to an infection and/or sinus formation.

To eliminate wire knotting and its problems, several types of connectors or locking implants (locking systems) have been developed to hold surgical wires, bands, cables etc. in desired position around and/or inside of bone. Common forms of locking members and systems are e.g. crimps (e.g. US Pat. No. 5,536,270, US Pat. No. 5,649,927), connectors (US Pat. No. 5,415,658), cable ties (US Pat. No. Des. 369,960, US Pat. No, 3,886,630), loop locking structures (US Pat. No. 4,813,416), suture locks (US Pat. No. 5,364,407), clamps (US Pat. No. 4,201,215), buckles (US Pat. No. 5,355,913) and locking systems comprising bioabsorbable fasteners (US Pat. No. 7,648,504).

However, current designs of (especially metallic) locking members and systems, particularly for surgical use, have significant drawbacks. For example, a tension which is too high may lead to improper healing or poor medical results (such as a bone fracture and/or necrosis).

Also, in many prior art locking systems there is the risk of the slippage of the wire or cable in relation to the locking member, which can lead to delayed healing. The loosening of the cable, e.g. in bone fracture fixation, can also lead to delayed healing, pain or even to a failure to heal. Also, many prior art locking members cannot be locked easily at a desired tension, and often the retained tension cannot be maintained when using a cable loop or winding as there is an inevitable drop in the tension when the pliers are removed. Thus, a surgeon typically has to "overshoot" the desired tension, approximating how much of that tension will be lost after the locking has been completed and the pliers have been removed thus significantly increasing the risk of tensioning errors.

In addition, many prior art locking members are bulky and may cause adverse tensioning in the surrounding tissues, which may result in a negative effect on tissue healing. Another drawback of many prior art cable and locking member systems is that they are made of metal, such as stainless steel. Such extremely stiff materials are mechanically incompatible with bone tissue and therefore, they may cause osteolysis around the material, which may lead to implant migration.

Consequently, it is desirable to use less stiff band and locking member systems, thereby preventing osteolysis and implant migration. It may also be desirable to use bands and locking member systems in which band slippage in relation to the locking member(s) is not possible. It may also be desirable to use bioabsorbable band and locking member systems so that the implant will absorb after healing of the bone fracture or osteotomy.

US Pat. No. 7,648,504 describes locking systems comprising bioabsorbable fasteners. However, drill holes must be drilled into the bone to fix the fasteners and the bioabsorbable band together. The drilling of additional drill holes into the bone creates an additional trauma to the patient and prolongs the duration of the surgical operation.

Publication WO 99/51159 shows a bioabsorbable surgical tissue fixation implant for the fixation of soft tissue to bone, comprising an oriented or self-reinforcing structure. In manufacturing the implant, a rod is bent at the end once with the help of heat to form a hook-like end portion. External shapes helping to retain the implant in a drill hole are made by machining in the straight shaft portion.

Therefore, there is still a need for a minimally invasive fixation device which is simple, easy to make and rapid and easy to use surgically. There is also a need for a fixation device in which the locking system is small but creates a secure locking and fixation of bone fragment(s).

### SUMMARY OF THE INVENTION

The present invention provides a bioabsorbable elongated member which provides accurate tensioning of the band and safe locking of the band around and/or inside of the bone by means of minimally invasive surgery.

The present invention also provides a bioabsorbable elongated member which is simple, easy to make and rapid and easy to use surgically.

The present invention also provides a bioabsorbable elongated member which is small but creates a secure locking of the member and fixed bone fragments.

The orientation of the elongated member of the invention is locally alterable, so that the elongated member is radially expandable. The radial expansion is a consequence of the microstructure of the implant of the invention. At least a part of the elongated member is constructed of a material having uniaxial, longitudinal stresses, formed therein by solid state drawing or pullforming. Multiaxial stresses are also possible. The multiaxial stresses are created for example by turning spirally a blank of the elongated member. These stresses in the material, when treated mechanically (such as by locally moving around and bending) are relieved, whereby a radial, local expansion of the material takes place, thus creating a bulge.

The property of creating the bulge can be identified simply by bending the bioabsorbable elongated member by hand repeatedly at the same point. The material of the bioabsorbable elongated member heats up due to the internal friction while it is bent. After a few bends, the bulge forms due to the relaxation of the material. Shortly after the bulge has formed, the bioabsorbable elongated member can be bent at the bulge in a desired direction, or the bulge can be left as such in order to form a local stopper, or a part of a local stopper. It is naturally also possible to move the elongated member in other ways than by bending; all mechanical treatments, for example rotating, having the same result, i.e. the bulge, are usable as well.

The main benefit of the mechanical treatment compared to the other possible methods is that the bulge can be formed in-situ during a surgical operation without special tools, i.e. one can freely decide where to form the bulge, and it can be done by hand simply for example by bending the elongated member back and forth at the same point. The bulge may be used as a stopper, a part of a stopper, or a bending point of a bioabsorbable member. The bulge may be used instead of knots, or other stoppers or locking members.

It is possible to use a tightening ring, a retaining plate, or a cannulated screw to secure that the bulge remains in its position if there is a risk that the elongated member may move.

A bioabsorbable member for securing a bone fracture, or bone fractures, is provided, comprising a first end, an elongated part and a second end. The elongated part comprises a front end, a back end and a target point in which the front end terminates and the back end begins. The bioabsorbable elongated member possesses a predetermined orientation. According to the invention, the bioabsorbable elongated member is radially expandable and comprises a bulge formed in the bioabsorbable elongated member by moving the front end and the back end repeatedly with regard to each other at the target point, thus relieving the stresses in the material of the bioabsorbable elongated member, said bulge created by radial loal expansion of the material forming a local stopper of the bioabsorbable elongated member, or a part of a local stopper, or a bending point of the bioabsorbable elongated member.

A method for forming a local stopper, a part of a local stopper, or a bending point in a bioabsorbable elongated member comprising a front end and a back end is also provided. According to the invention, the method comprises
- determining a target point where the front end terminates and the back end begins, and
- relieving the stresses in the material of the bioabsorbable elongated member so that a bulge is formed in the bioabsorbable elongated member by moving the front end and the back end repeatedly with regard to each other at the target point, thereby forming by radial local expansion of the material a bulge used as the local stopper, or the part of the local stopper, or the bending point.

The bioabsorbable implants (bioabsorbable bands or rods) of this invention may be manufactured of bioabsorbable polymers, copolymers or polymer mixtures or alloys. The preferred material is a copolymer of lactide and glycolide. The material may comprise 70 to 85 wt.-% lactide and 15 to 30 wt.-% glycolide. Suitable manufacturing methods include molding, sintering and/or solid state deformation (pullforming) methods which are described e.g. in US Pat. No. 4,743,257, US Pat. No. 4,968,317, EP Pat. No. 0423155, AU Pat. No. 729801, EP Pat. No. 1009448 and in US Pat. No. 6,406,598.

The strong and tough oriented structures are especially advantageous in implant systems of this invention. They may be created also during extrusion or injection molding of absorbable polymeric melt through a suitable die or into a suitable mold at high speed and pressure. When cooling occurs at suitable conditions, the flow orientation of the melt may remain in the solid material as an oriented structure. In an advantageous embodiment, the mold may have the form of the final device, but it is also possible to manufacture the implants of the invention by machining (possibly using also heat) and by thermoforming of injection-molded or extruded semi-finished products.

It is advantageous to make the implants of melt-molded, solid state drawn or compressed, bioabsorbable polymeric materials, which are described e.g. in United States Patent No. 4,968,317 or 4,898,186.

The reinforcing fibers of the implants may also be ceramic fibers, such as bioabsorbable hydroxyapatite or bioactive glass or tricalcium phosphate fibers. Such bioabsorbable, ceramic fiber reinforced materials are described e.g. in European Patent Application No. 0146398 and in WO 96/21628.

The oriented and/or fiber reinforced implants of this invention may be manufactured by molding the reinforcement fiber-polymer matrix to the final product in a mold whose mold cavity has the form of the final product, or the final form may be machined mechanically (possibly also using heat) of a preform, such as a melt-molded and solid-state drawn rod, as described e.g. in United States Patent No. 4,968,317.

The reinforcement elements may extend into any protrusions or ridges of the implant. The reinforcement elements may also turn spirally around the longitudinal axis of the implants. Also other different orientations of reinforcement elements in elongated samples which are known in composite technology may be applied to the present invention. However, a general feature of the orientation and/or fiber-reinforcement of the implants of this invention is that many of the reinforcing elements are oriented in such a way that they can carry effectively the different external loads (such as tensile, bending and shear loads) that are directed to the healing bone fracture or osteotomy, e.g. sternotomy.

According to an advantageous embodiment of the invention, the implant, or a special coating layer on its surface, may contain one or more bioactive substances, such as antibiotics, chemotherapeutic substances, growth factors such as bone morphogenic proteins, substances accelerating the healing of the wound and osteotomy, hormones, antibiotics or other drugs and the like. Such bioactive implants are especially advantageous in surgical use, because they contribute biochemically to the healing of the lesion in addition to providing mechanical support.

The oriented and/or reinforced materials of the implants of this invention typically have initial tensile strengths of about 100 to 1000 MPa, bending strengths of about 100 to 500 MPa and shear strengths of about 80 to 300 MPa. The implants can be made stiff, tough, and/or flexible. These mechanical properties are superior to those of non-oriented and non-reinforced absorbable polymers which typically show strengths between 40 and 100 MPa and may additionally be brittle (see e.g. S. Vainionpää, P. Rokkanen and P. Törmälä, "Surgical Applications of Biodegradable Polymers in Human Tissues", Progr. Polym. Sci 14/1989, pp. 679-716).

A special advantage of the present invention is that there are no bulky crimps in these implants. They can be made relatively thin e.g. with a cylindrical shaft part having a diameter between 0.5 and 4 mm and a bulge having a diameter between 1 and 8 mm.

The implants of the present invention may be sterilized by any of the well known sterilization techniques, depending on the type of material used in manufacture of the implant. Suitable sterilization techniques include radiation sterilization such as cobalt 60 irradiation or electron beams, ethylene oxide sterilization, and the like.

The elongated member may be provided with at least one needle. Instead of the needle there may also be a drill bit or a kirschner wire by which a drillhole can be drilled into a bone.

The possible uses of the elongated member comprise, for example, olecranon fractures, patella fractures, tuberculum majus fractures of proximal humerus, comminuted fractures of distal tibia (also syndesmosis) and comminuted proximal tibia fractures. In reconstructive plastic surgery the elongated member can be used for the fixation of bone containing a latissimus dorsi graft. The elongated member may be used in combination with other pins and screws.

### BRIEF DESCRIPTION OF THE DRAWINGS

In figures only Figs. 1b, 1c, 9e - 9i, 12b show a bone to be fixed. However, a person skilled in the art will readily understand how the bone or tissue exists with respect to an elongated member in other figures.

In the figures,
- Fig. 1a: shows an elongated member in a perspective view,
- Figs. 1b and 1c: show the elongated member inserted in a bone (the bone is shown in a cross-sectional view in order to show the elongated member,
- Figs. 2a to 8c: show variations of elongated members in perspective views,
- Figs. 9a to 9d: show an elongated member in a perspective view,
- Figs. 9e to 9j: show a schematical view how the elongated member of Fig. 9a is inserted in a cranium,
- Figs. 10a to 10d: show an elongated member in a side view,
- Figs. 11a to 11d: show possible head designs of an elongated member,
- Fig. 12a: shows fractures in the distal end of a tibia,
- Fig. 12b: shows the distal end of the tibia of Fig. 12a fixed with an elongated member,
- Figs. 13a to 13e: show one possible way to form a loop from an elongated member,
- Figs. 14a to 14c: show an elongated member with a retaining plate, and
- Fig. 15: shows a cross-sectional view of elongated members which are used with cannulated screws and a retaining plate.

### DETAILED DESCRIPTION OF THE INVENTION

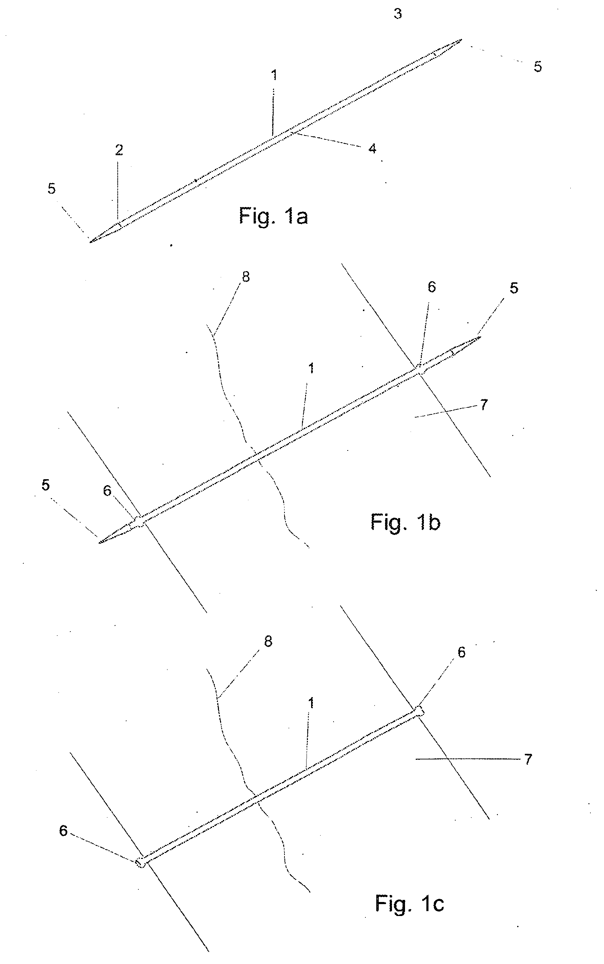

Fig. 1 a shows a bioabsorbable elongated member which is a rod comprising a shaft 1. The cross-section of the rod may be cylindrical, square or any other suitable cross-section. The shaft 1 comprises a first end 2 and a second end 3 and an elongated part 4 therebetween. Needles 5 assisting in penetrating into a bone or tissue are attached to the both ends 2, 3 of the shaft 1. It is also possible that there is only one needle in one end of the shaft 1. The rod is delivered to its user as it is illustrated in Fig. 1 a.

Fig. 1b shows the bioabsorbable elongated member, i.e. the rod of Fig. 1a, in such a situation in which the user has inserted the rod in a drillhole in a broken bone 7 (number 8 denotes the fracture) and he has formed two bulges 6 in the shaft 1. Providing both ends with needles 5 makes it easier to fix rather complicated fractures.

Fig. 1c shows the rod of Figs. 1a and 1b when the excess of the shaft 1 has been cut off.

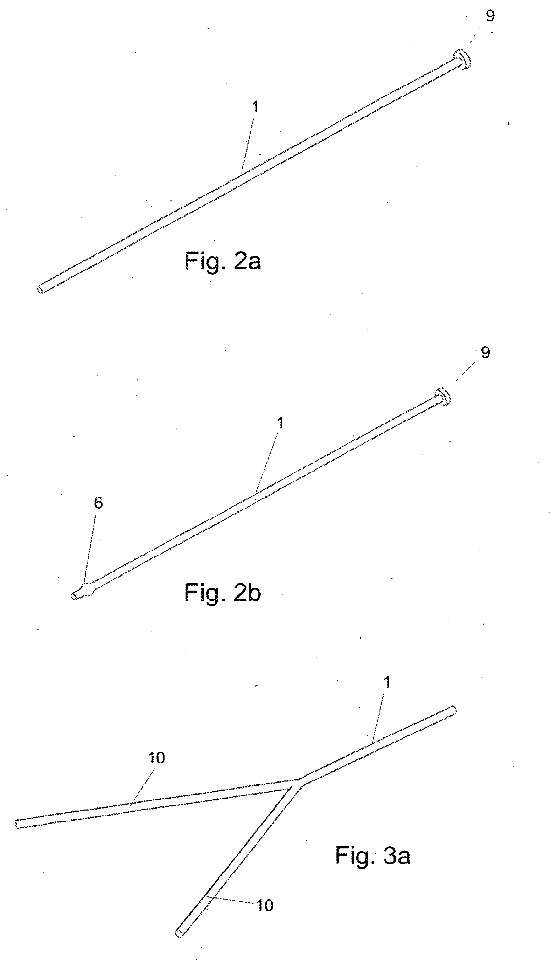

Fig. 2a shows a rod comprising a shaft 1 and a head 9. The head 9 is formed by pressing down. The rod is delivered to its user as it is illustrated in Fig. 2a.

Fig. 2b shows a situation in which the rod is inserted in a drillhole in a broken bone 7 (not shown). A bulge 6 forming a stopper is formed at the end of the shaft 1.

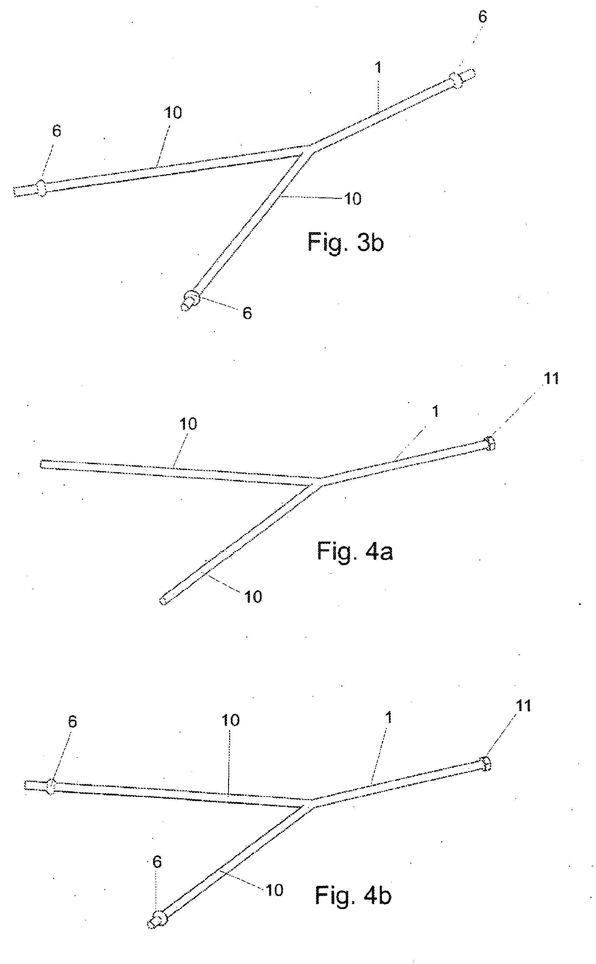

Fig. 3a shows an elongated member whose shaft 1 comprises branches 10. The branches 10 of the elongated member may be inserted in drillholes in a broken bone. After the branches have been positioned, bulges 6 are formed outside the bone 7 (not shown) as shown in Fig. 3b.

Fig. 4 shows another variation of the elongated member of Fig. 3. The elongated member comprises a pre-manufactured head 11 which may be manufactured by pressing down. Branches 10 are inserted in a drillhole in a bone and bulges 6 are formed in the branches.

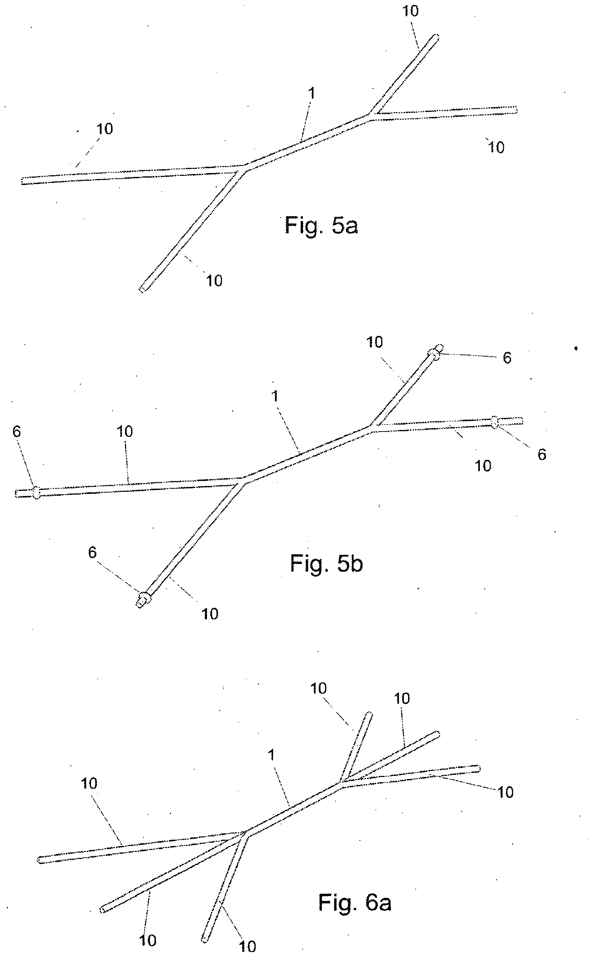

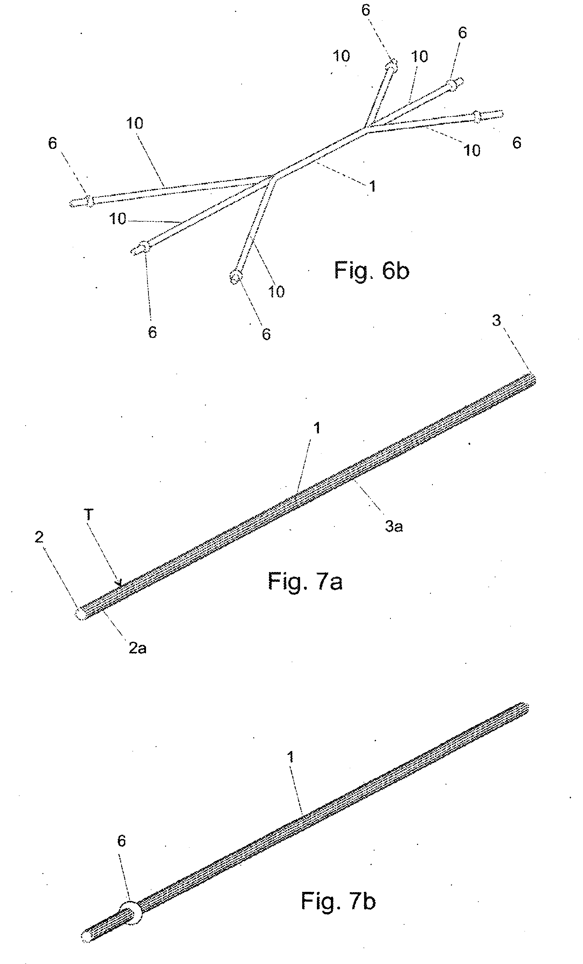

Figs. 5 and 6 also show branched elongated members. Figs. 5a and 6a show the elongated members before implanting, and Figs. 5b and 6b show the elongated members after implanting. Bulges 6 are formed in each branch.

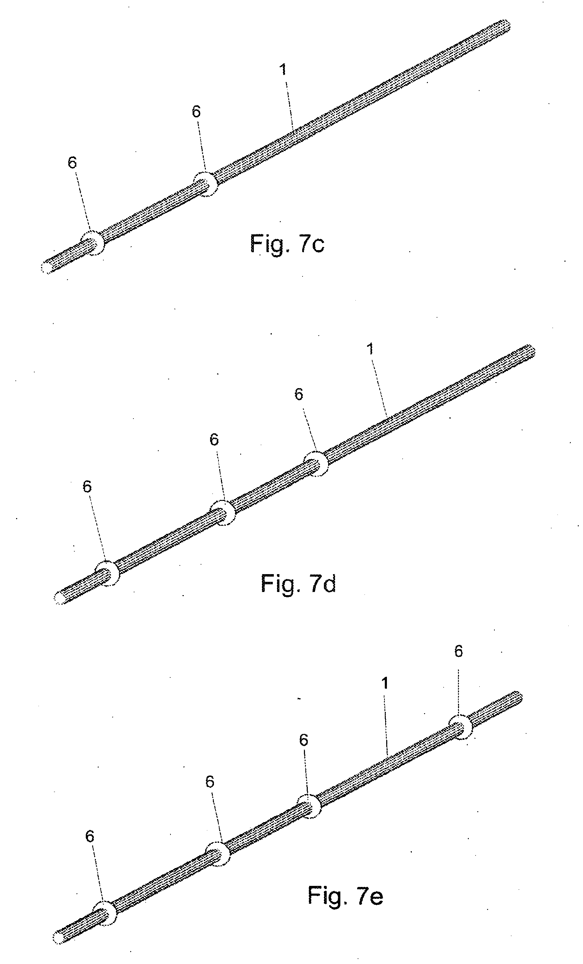

Fig. 7a shows an elongated member, such as a pin which has a grooved surface. Fig. 7a also shows a first end 2, a second end 3, a front end 2a, a back end 3a and a target point T. The front end 2a begins from the first end 2 and terminates at the target point T. The back end 3a begins from the target point T and terminates at the second end 3. A bulge 6 is formed at the target point T. Figs. 7b to 7e show that in principle a bulge 6 can be formed in the pin wherever desired, i.e. there can be several target points T.

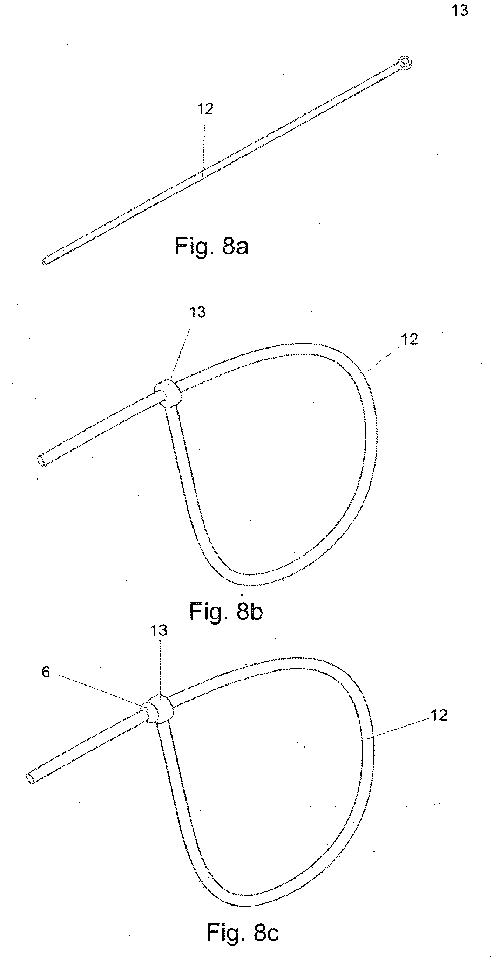

Fig. 8a shows an elongated member whose outer appearance resembles a cable tie. The elongated member has a flexible band section 12. The elongated member also comprises in its one end an eyelet 13 in which the other end can be threaded, as shown in Fig. 8b. After the elongated member has been inserted in a bone or tissue in a desired manner and threaded, a bulge 6 is formed outside the eyelet. Thus, the bulge forms a stopper as shown in Fig. 8c.

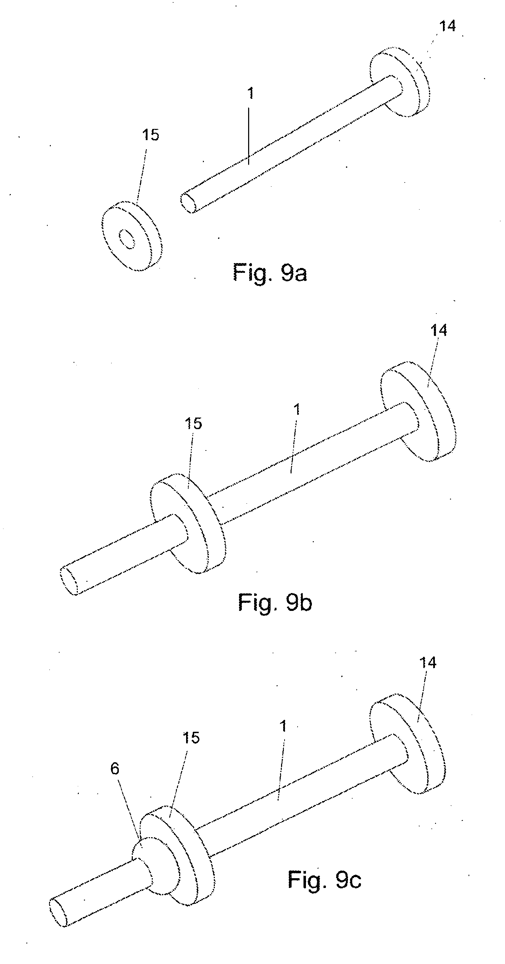

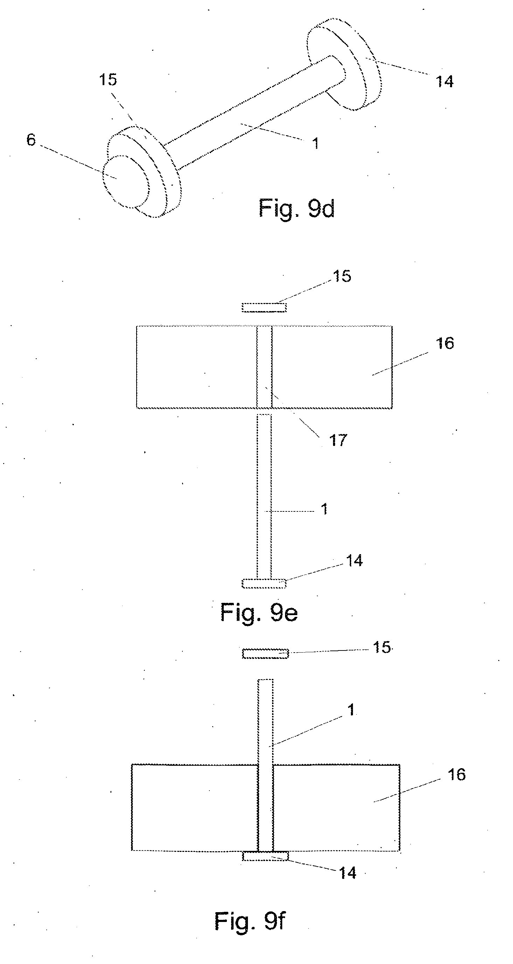

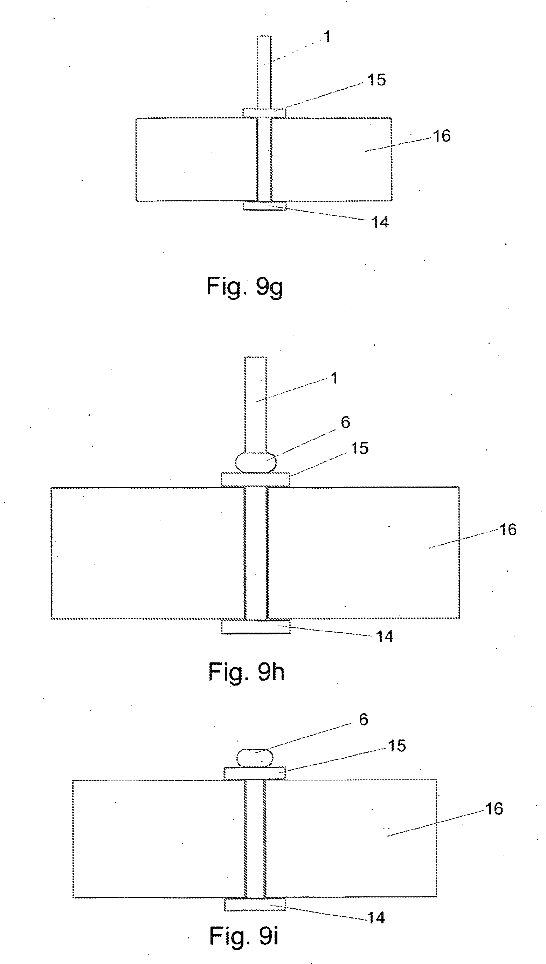

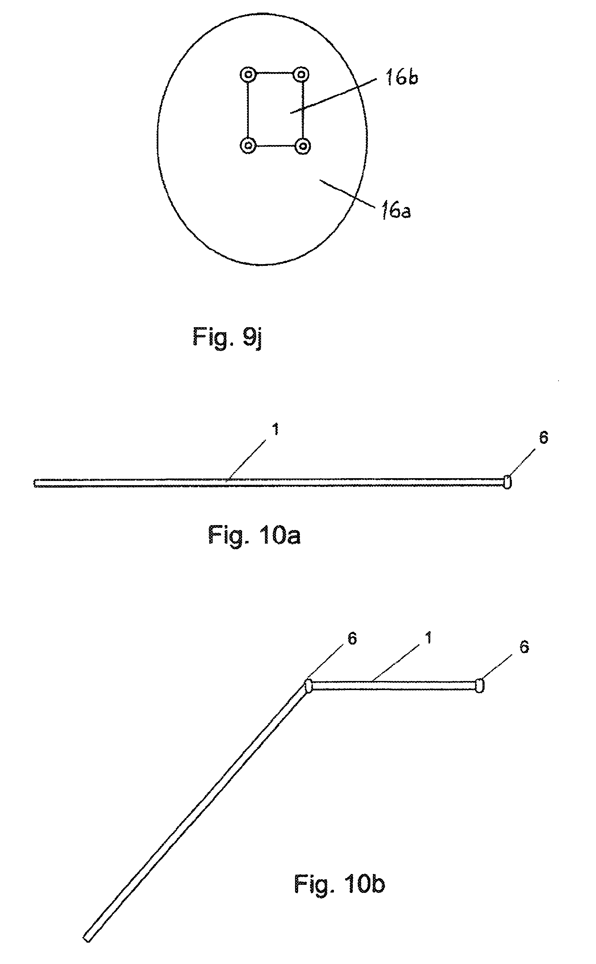

Fig. 9 shows an elongated member which is used e.g. in applications in which the cranium is fixed. The elongated member comprises a shaft 1 and a head 14. The shaft 1 of the elongated member is inserted through a drillhole in the cranium so that the head 14 of the elongated member is left under the cranium but over the dura. The head of the elongated member is a flat round plate as is natural due to the application. The diameter of the round plate may be at least 10 mm. The diameter of the shaft 1 may be around 10 mm.

After the elongated member has been inserted into the drillhole 17, the shaft 1 protrudes outside the cranium 16. An annular flat tightening ring 15 is inserted to the shaft 1, and a bulge 6 is formed outside the tightening ring 15, as shown in Figs. 9c and 9h. The rest of the shaft 1 is cut as shown in Figs. 9d and 9i.

Fig. 9j shows the cranium, which is fixed with the elongated members, from above. A piece of the cranial bone 16b has been separated from the rest of the cranium 16a in order to make surgical operations. There are four drillholes in each corner of the piece of the cranial bone in which elongated members are inserted. The elongated members are fastened at the edge of the piece of the cranial bone 16b. Bulges are formed on the elongated member so that they keep the piece of the cranial bone 16b in its position (see Fig. 9i).

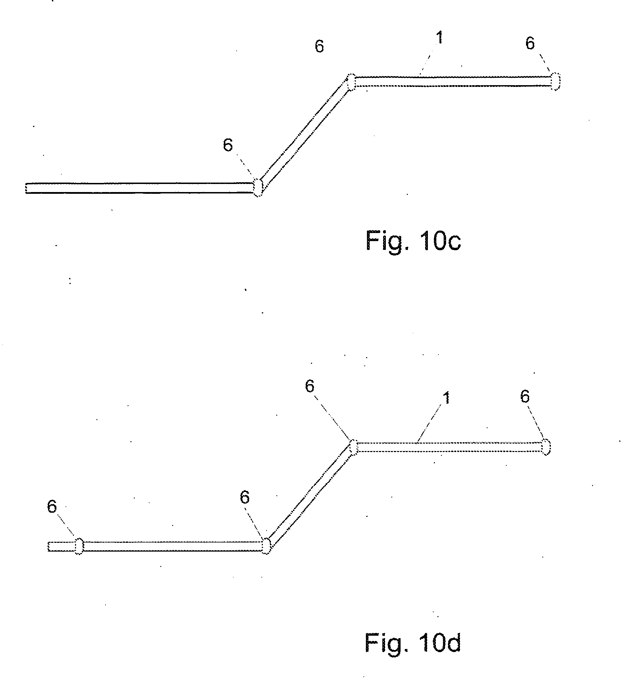

Fig. 10 shows an elongated member which is useful in applications in which the elongated member shall change its advancing direction. The elongated member may comprise a prefabricated head, or the head may be formed in situ. The advancing direction of the elongated member may be changed as desired as shown in Figs. 10b and 10c by mechanically straining a certain point of the elongated member. When the material deforms at the point due to the heat caused by the mechanical straining, it is possible to form an angle in the elongated member. Fig. 10d shows a situation in which a desired length of the elongated member is used for the fixation and a bulge is formed at the end of the elongated member.

It is also possible to bend the elongated member without making the bulge 6 if the elongated member is sufficiently flexible. However, the bulge 6 increases the strength of the elongated member in its bending direction.

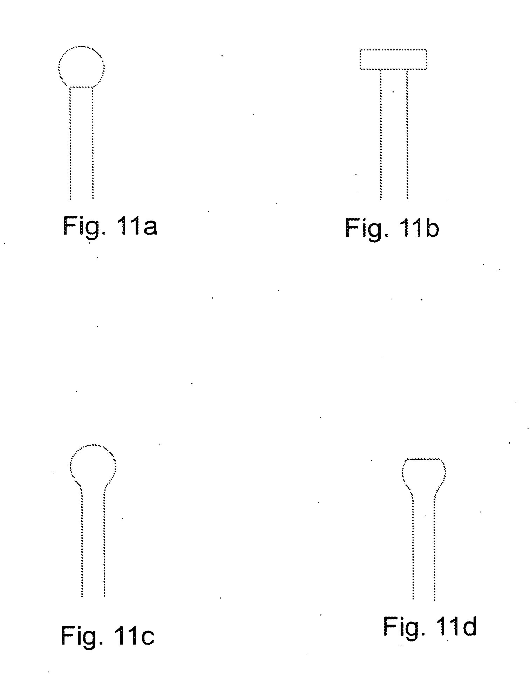

Fig. 11 shows variations of head designs of an elongated member. Fig. 11 a shows a spherical head, Fig. 11b shows a flat round head, and Fig. 11c shows a spherical head whose joint with the shaft has been made gentle. Fig. 11d shows a truncated spherical head whose joint with the shaft has been made gentle.

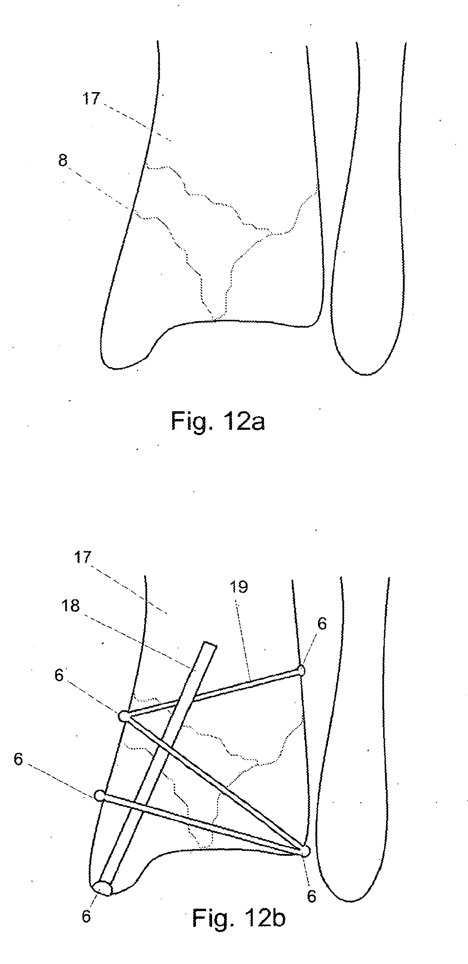

Fig. 12a shows fractures 8 in the distal end of tibia 17. Fig. 12b shows how the fractures 8 have been fixed with elongated members 18 and 19. The elongated member 18 is a pin which has been inserted in drillholes penetrating through the tibia 17. The elongated member 19 is a rod which has been inserted in a drillhole and whose advancing direction has been changed by forming bulges 6 outside the tibia 17.

The tibia is an example about possible targets of the elongated member. The principle of fixation is also the same with fractures in other bones: The elongated member is inserted in a drillhole or drillholes in a bone, and bulges are formed in the elongated member outside the bone in order to use them as stoppers or bending points.

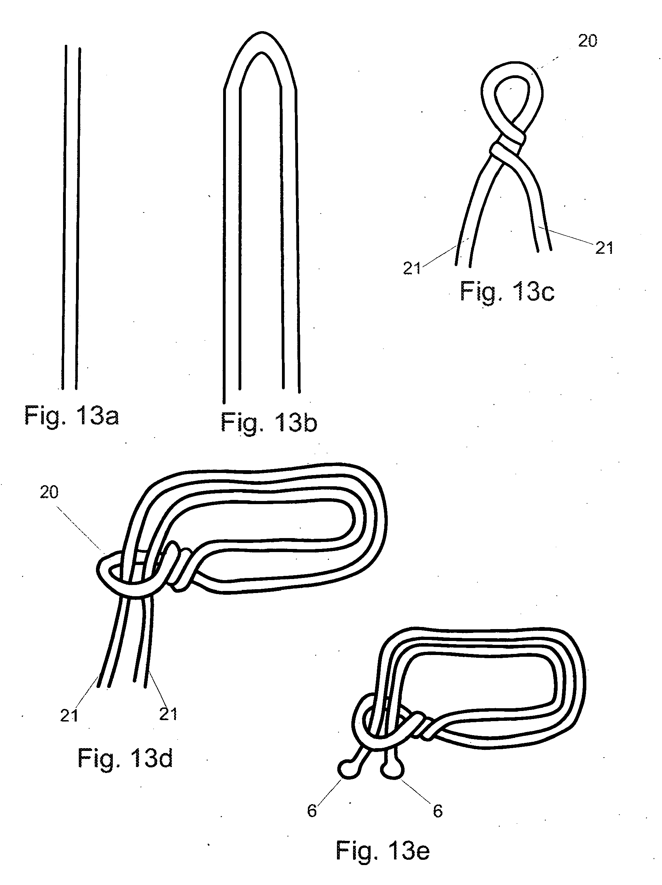

Figs. 13a to 13e show one possible way to form a loop 20 from an elongated member. The straight elongated member of Fig. 13a is bent (Fig. 13b) and twisted (Fig. 13c) so that the loop 20 and two elongated tails 21 are formed. The legs 21 of the elongated member are threaded into the loop 20 (Fig. 13d). Bulges 6 are formed on the tails 21, and the tails 21 are cut underneath the bulges 6. The bulges 6 prevent the tails 21 from slipping out the loop 20.

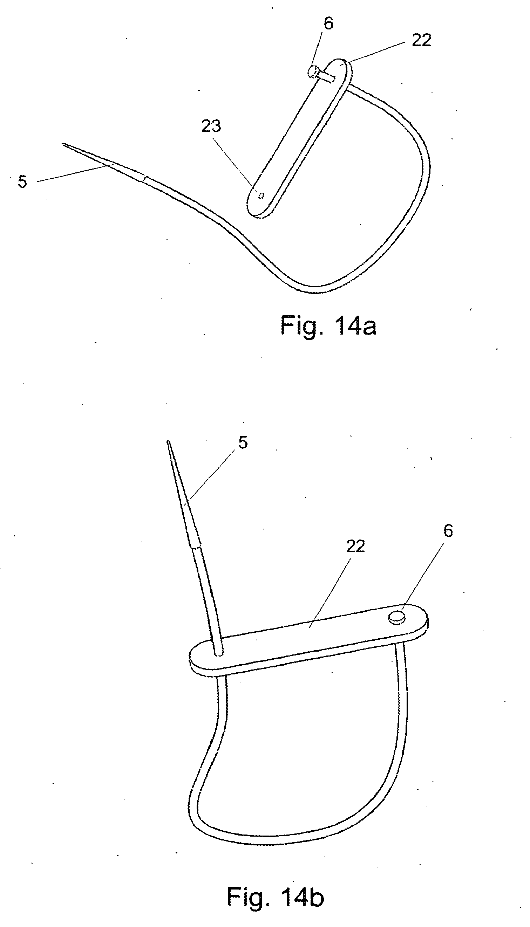

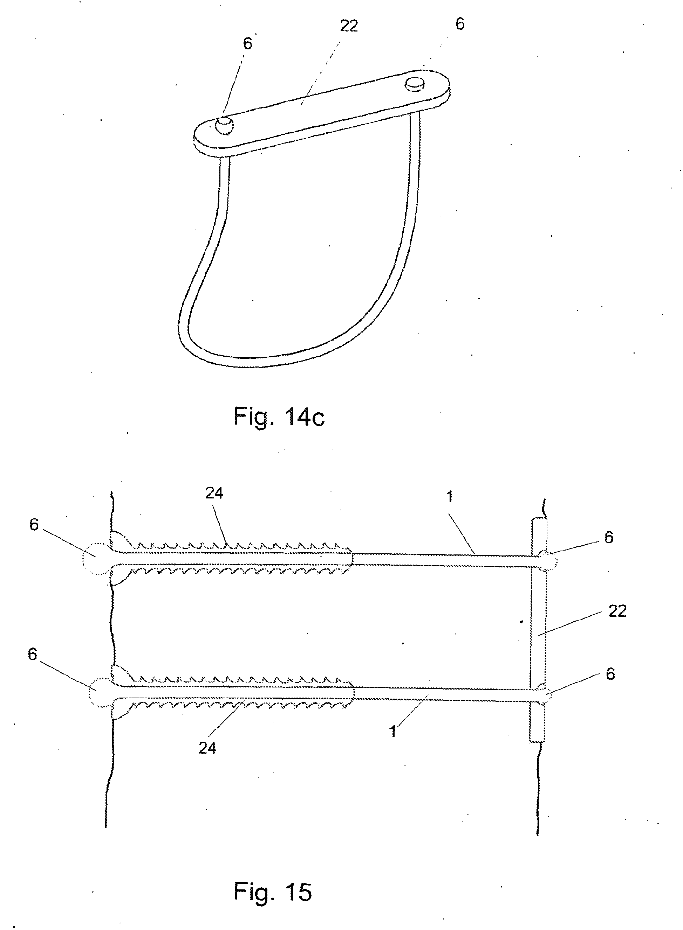

Figs. 14a to 14c show an elongated member with a retaining plate 22. It is possible to form a bulge 6 on the elongated member before inserting the elongated member into a bone and/or tissue, or the bulge 6 can be formed after inserting the elongated member. The elongated member may be provided with a needle 5. After the elongated member has been inserted it forms either a loop around the bone and/or tissue, or it has been inserted in a drillhole or drillholes in the bone. It can be used for example for the fixation of the sternum. The ends of the elongated member are threaded through holes 23 in the retaining plate 22 and they are provided with the bulges 6. The elongated member is cut from the side of the bulge 6. The retaining plate 22 secures that the bulges 6 are prevented from slipping out from their position.

Fig. 15 shows elongated members which are used with cannulated screws 24 and a retaining plate 22. The cannulated screws 24 have been inserted in a bone and the elongated members are inserted via drillholes through the cannulated screws 24 and holes in the retaining plate 22. The ends of the elongated members are provided with bulges 6 which act as local stoppers. The cannulated screws 24 and the retaining plate 22 secures that the bulges 6 do not slip into the drillholes.

On the basis of the above description of the present invention and certain specific embodiments thereof, it will be readily apparent to those skilled in the art that many variations and modifications may be made to the present invention according to the following claims.

## Claims

1. A bioabsorbable elongated member comprising a first end (2) and a second end (3) and an elongated part therebetween, the elongated part comprising a front end (2a), a back end (3a) and a target point (T) in which the front end (2a) terminates and the back end (3a) begins, the bioabsorbable elongated member possessing a predetermined orientation, **characterized in that** the bioabsorbable elongated member is radially expandable and comprises a bulge (6) formed in the bioabsorbable elongated member by moving the front end (2a) and the back end (3a) repeatedly with regard to each other at the target point (T), thus relieving the stresses in the material of the bioabsorbable elongated member, said bulge (6) created by radial local expansion of the material forming a local stopper of the bioabsorbable elongated member, or a part of a local stopper, or a bending point of the bioabsorbable elongated member.

2. The bioabsorbable elongated member according to any preceding claim, **characterized in that** it comprises a shaft (1).

3. The bioabsorbable elongated member according to claim 1 or 2, **characterized in that** the bioabsorbable elongated member comprises branches (10).

4. The bioabsorbable elongated member according to claim 1, **characterized in that** it comprises a band (12).

5. The bioabsorbable elongated member according to claim 4, **characterized in that** the band (12) comprises a loop (13) at one end of the band.

6. The bioabsorbable elongated member according to any preceding claim, **characterized in that** the bioabsorbable elongated member comprises a separate tightening ring (15) or a separate retaining plate (22).

7. The bioabsorbable elongated member according to any preceding claim, **characterized in that** the bioabsorbable elongated member comprises a needle (5) or a bore bit.

8. The bioabsorbable elongated member according to any preceding claim, **characterized in that** it is a copolymer of lactide and glycolide.

9. The bioabsorbable elongated member according to claim 8, **characterized in that** it comprises 70 to 85 wt.-% of lactide and 15 to 30 wt.-% glycolide.

10. A method for forming a local stopper, a part of a local stopper, or a bending point in a bioabsorbable elongated member comprising a front end and a back end, the method comprising:
- determining a target point where the front end terminates and the back end begins, and **characterized in that** the method further comprises
- relieving the stresses in the material of the bioabsorbable elongated member so that a bulge is formed in the bioabsorbable elongated member by moving the front end and the back end repeatedly with regard to each other at the target point (T), thereby forming by radial local expansion of the material said bulge (6) used as the local stopper, or the part of the local stopper, or the bending point.

11. The method according to claim 10, **characterized in that** the bulge is formed by locally rotating or bending the elongated member.

12. The method according to claim 10 or 11, **characterized in that** before forming the bulge (6), a tightening ring (15) or a retaining plate (22) is inserted on the bioabsorbable elongated member.

13. The method according to any preceding claim, **characterized in that** after forming the bulge (6), the excess of the bioabsorbable elongated member is cut and/or pressed down.

## Patentansprüche

1. Verlängertes bioabsorbierbares Element, aufweisend ein erstes Ende (2) und ein zweites Ende (3) und ein verlängertes Teil dazwischen, wobei das verlängerte Teil ein vorderes Ende (2a), ein hinteres Ende (3a) und einen Zielpunkt (T) aufweist, in dem das vordere Ende (2a) endet und das hintere Ende (3a) beginnt, wobei das verlängerte bioabsorbierbare Element eine vorbestimmte Ausrichtung aufweist, **dadurch gekennzeichnet, dass** das verlängerte bioabsorbierbare Element radial ausdehnbar ist und eine Ausbauchung (6) aufweist, die in dem verlängerten bioabsorbierbaren Element gebildet wird, indem das vordere Ende (2a) und das hintere Ende (3a) wiederholt an dem Zielpunkt (T) zueinander bewegt werden, wodurch die Spannungen in dem Material des verlängerten bioabsorbierbaren Elements gelöst werden, wobei die Ausbauchung (6), die durch eine radiale lokale Ausdehnung des Materials gebildet wird, einen lokalen Anschlag des verlängerten bioabsorbierbaren Elements oder einen Teil eines lokalen Anschlags oder einen Biegepunkt des verlängerten bioabsorbierbaren Elements bildet.

2. Verlängertes bioabsorbierbares Element nach einem der vorhergehenden Ansprüche, **dadurch gekennzeichnet, dass** es einen Schaft (1) aufweist.

3. Verlängertes bioabsorbierbares Element nach Anspruch 1 oder 2, **dadurch gekennzeichnet, dass** das verlängerte bioabsorbierbare Element Verzweigungen (10) aufweist.

4. Verlängertes bioabsorbierbares Element nach Anspruch 1, **dadurch gekennzeichnet, dass** es ein Band (12) aufweist.

5. Verlängertes bioabsorbierbares Element nach Anspruch 4, **dadurch gekennzeichnet, dass** das Band (12) eine Schlaufe (13) an einem Ende des Bands aufweist.

6. Verlängertes bioabsorbierbares Element nach einem der vorhergehenden Ansprüche, **dadurch gekennzeichnet, dass** das verlängerte bioabsorbierbare Element einen separaten Spannring (15) oder eine separate Halteplatte (22) aufweist.

7. Verlängertes bioabsorbierbares Element nach einem der vorhergehenden Ansprüche, **dadurch gekennzeichnet, dass** das verlängerte bioabsorbierbare Element eine Nadel (5) oder einen Bohreinsatz aufweist.

8. Verlängertes bioabsorbierbares Element nach einem der vorhergehenden Ansprüche, **dadurch gekennzeichnet, dass** es ein Copolymer aus Laktid und Glycolid ist.

9. Verlängertes bioabsorbierbares Element nach Anspruch 8, **dadurch gekennzeichnet, dass** es 70 bis 85 Gew.-% Laktid und 15 bis 30 Gew.-% Glycolid aufweist.

10. Verfahren zum Bilden eines lokalen Anschlags, eines Teils eines lokalen Anschlags oder eines Biegepunkts in einem verlängerten bioabsorbierbaren Element, aufweisend ein vorderes Ende und ein hinteres Ende, wobei das Verfahren aufweist:
- das Bestimmen eines Zielpunktes, an dem das vordere Ende endet und das hintere Ende beginnt, und **dadurch gekennzeichnet, dass** das Verfahren ferner aufweist:
- Lösen der Spannungen in dem Material des verlängerten bioabsorbierbaren Elements, so dass eine Ausbauchung in dem verlängerten bioabsorbierbaren Element gebildet wird, indem das vordere Ende und das hintere Ende an dem Zielpunkt (T) wiederholt zueinander bewegt werden, wodurch durch radiale lokale Ausdehnung des Materials die Ausbauchung (6), die als lokaler Anschlag verwendet wird, oder der Teil des lokalen Anschlags oder der Biegepunkt gebildet wird.

11. Verfahren nach Anspruch 10, **dadurch gekennzeichnet, dass** die Ausbauchung durch lokales Drehen oder Biegen des verlängerten Elements gebildet wird.

12. Verfahren nach Anspruch 10 oder 11, **dadurch gekennzeichnet, dass** vor Bildung der Ausbauchung (6) ein Spannring (15) oder eine Halteplatte (22) auf dem verlängerten bioabsorbierbaren Element eingeschoben wird.

13. Verfahren nach einem der vorhergehenden Ansprüche, **dadurch gekennzeichnet, dass** nach Bildung der Ausbauchung (6) der Überschuss des verlängerten bioabsorbierbaren Elements abgeschnitten und/oder heruntergedrückt wird.

## Revendications

1. Élément allongé bioabsorbable comprenant une première extrémité (2) et une deuxième extrémité (3) et une partie allongée entre celle-ci, la partie allongée comprenant une extrémité avant (2a), une extrémité arrière (3a) et un point cible (T) dans lequel l'extrémité avant (2a) se termine et l'extrémité arrière (3a) commence, l'élément allongé bioabsorbable possédant une orientation prédéterminée, **caractérisé en ce que** l'élément allongé bioabsorbable est radialement extensible et comprend un renflement (6) formé dans l'élément allongé bioabsorbable en déplaçant l'extrémité avant (2a) et l'extrémité arrière (3a) de façon répétée l'une par rapport à l'autre au niveau du point cible (T), libérant ainsi les contraintes dans le matériau de l'élément allongé bioabsorbable, ledit renflement (6) créé par expansion locale radiale du matériau formant une butée locale de l'élément allongé bioabsorbable, ou une partie d'une butée locale, ou un point de flexion de l'élément allongé bioabsorbable.

2. Élément allongé bioabsorbable selon une quelconque revendication précédente, **caractérisé en ce qu'**il comprend un axe (1).

3. Élément allongé bioabsorbable selon la revendication 1 ou 2, **caractérisé en ce que** l'élément allongé bioabsorbable comprend des ramifications (10).

4. Élément allongé bioabsorbable selon la revendication 1, **caractérisé en ce qu'**il comprend une sangle (12).

5. Élément allongé bioabsorbable selon la revendication 4, **caractérisé en ce que** la sangle (12) comprend une boucle (13) à une extrémité de la sangle.

6. Élément allongé bioabsorbable selon une quelconque revendication précédente, **caractérisé en ce que** l'élément allongé bioabsorbable comprend un anneau de serrage (15) séparé ou une plaque de maintien (22) séparée.

7. Élément allongé bioabsorbable selon une quelconque revendication précédente, **caractérisé en ce que** l'élément allongé bioabsorbable comprend une aiguille (5) ou une mèche.

8. Élément allongé bioabsorbable selon une quelconque revendication précédente, **caractérisé en ce qu'**il s'agit d'un copolymère de lactide et de glycolide.

9. Élément allongé bioabsorbable selon la revendication 8, **caractérisé en ce qu'**il comprend 70 à 85% en poids de lactide et 15 à 30% en poids de glycolide.

10. Procédé de formation d'une butée locale, d'une partie de butée locale, ou d'un point de flexion dans un élément allongé bioabsorbable comprenant une extrémité avant et une extrémité arrière, le procédé comprenant :
- la détermination d'un point cible où l'extrémité avant se termine et l'extrémité arrière commence, et
**caractérisé en ce que** le procédé comprend en outre
- la libération des contraintes dans le matériau de l'élément allongé bioabsorbable de façon à ce qu'un renflement soit formé dans l'élément allongé bioabsorbable en déplaçant l'extrémité avant et l'extrémité arrière de façon répétée l'une par rapport à l'autre au niveau du point cible (T), formant ainsi par expansion locale radiale du matériau ledit renflement (6) utilisé comme la butée locale, ou la partie de la butée locale, ou le point de flexion.

11. Procédé selon la revendication 10, **caractérisé en ce que** le renflement est formé par rotation ou flexion locale de l'élément allongé.

12. Procédé selon la revendication 10 ou 11, **caractérisé en ce qu'**avant la formation du renflement (6), un anneau de serrage (15) ou une plaque de maintien (22) est inséré(e) dans l'élément allongé bioabsorbable.

13. Procédé selon une quelconque revendication précédente, **caractérisé en ce qu'**après la formation du renflement (6), l'excès de l'élément allongé bioabsorbable est coupé et/ou écrasé.
